# NEUE EUROPÄISCHE PATENTSCHRIFT

(11) **EP 0 108 248 B2**
(45) Veröffentlichungstag und Bekanntmachung des Hinweises auf die Entscheidung über den Einspruch: **15.09.1999**
(45) Hinweis auf die Patenterteilung: 28.12.1988
(21) Anmeldenummer: 83109887.6
(22) Anmeldetag: 04.10.1983
(51) Int. Cl.: A61K 9/00, A61K 31/21

(54) **Lösungsvermittlerfreie, wässrige Nitroglycerinlösung**
Aqueous nitroglycerine solution free of solubilizers
Solution aqueuse de nitroglycérine exempte de solubilisateurs

(30) Priorität: 08.10.1982 DE 3237284
(43) Veröffentlichungstag der Anmeldung: 16.05.1984
(73) Patentinhaber: G. Pohl-Boskamp GmbH & Co. Chemisch-pharmazeutische Fabrik, 25551 Hohenlockstedt (DE)
(72) Erfinder: Burghart, Kurt, D-2211 Rosdorf (DE)
(74) Vertreter: UEXKÜLL & STOLBERG

(56) Entgegenhaltungen:
- CHEMICAL ABSTRACTS, Band 84, Nr. 2, 12. Januar 1976, Seiten 258,259, Nr. 8908g, Columbus, Ohio, USA
- The Merck Index 1976, pages 858-859, no. 6429
- CHEMICAL ABSTRACTS, Band 92, Nr. 20, 19. Mai 1980, Seite 339, Nr. 169140f, Columbus, Ohio, USA
- Journal of Pharmaceutical Sciences, Band 71 [1], Januar 1982, Seiten 55 bis 59
- American Heart Journal, Band 96 [4], 1978, Seiten 550 bis 553
- Hunnius, Pharmazeutisches Wörterbuch, 6. Auflage, S. 648, Seiten 210-211
- Römpp's Chemielexikon, 7. Auflage, S. 1994
- Herbert P. Fiedler, Lexikon der Hilfsstoffe für Pharmazie, Kosmetik und angrenzende Gebiete, 4. Auflage, Band 2 [L-Z], Seiten 1278 - 1282

## Beschreibung

Die Erfindung betrifft ein Verfahren zur Herstellung einer Nitroglycerinlösung, die frei von Lösungsvermittlern und organischen Lösungsmitteln ist.

Nitroglycerinlösungen mit einer Konzentration von etwa 1 mg Nitroglycerin/1 ml Lösung bieten den Vorteil, dass sie eine leichte Berechnungsgrundlage für infundierte Mengen und weitere Verdünnungen bieten. Ein weiterer Vorteil besteht darin, dass Patienten mit geringen Infusionsvolumina belastet werden und Infusionskolbenpumpen bei Intensivbehandlungen selten gewechselt werden müssen.

Bekannte Nitroglycerinlösungen besitzen jedoch Nitroglycerinkonzentrationen von sehr viel weniger als 1 mg/ml Lösung oder zeigen einen toxikologisch unerwünscht hohen Gehalt an organischen Lösungsmitteln und/oder einen hypertonischen Wert, d.h. sie sind nicht isotonisch. Eine ausführliche Übersicht über den Stand der Technik findet sich in der DE-A-31 09 793, auf die hiermit ausdrücklich Bezug genommen wird.

Zur Vermeidung der mit den Nitroglycerinlösungen gemäss dem Stand der Technik verbundenen Probleme wird in der zuvor genannten Patentanmeldung vorgeschlagen, zur Herstellung von etwa isotonischen wässrigen Nitroglycerinlösungen im technischen Massstab eine Zubereitung in Wasser aufzulösen, die neben Nitroglycerin und einem für isotonische Grundlösungen üblichen, ihnen Isotonie verleihenden Stoff als festem Nitroglycerinträger einen für parenterale Applikationen üblichen Lösungsvermittler enthält. Wenngleich durch diesen Vorschlag, durch den insbesondere die Verwendung von organischen Lösungsmitteln vermieden wird, ein grosser Teil der mit den bekannten Nitroglycerinlösungen verbundenen Pro bleme gelöst wird, besteht weiterhin die Schwierigkeit, dass die vorgeschlagenen Nitroglycerinlösungen unerwünschte Zusatzstoffe enthalten, die aus medizinischer Sicht nicht unbedenklich sind. Es sei hierzu auf «Deutsche Medizinische Wochenschrift» 1982, Seiten 48 bis 45 und Seiten 51 bis 54 und «Pharmazeutische Technologie», 1978, Georg Thieme Verlag Stuttgart, Seite 551, verwiesen, wonach für parenterale Applikationen üblichen Lösungsvermittler wie Polyvinylpyrrolidon, Dimethylformamid, Tetraethylharnstoff, Isopropylenglykolether, Diethylglykoldimethylether, Tetrahydrofurfurylmethylether, N,N-Dimethylmethoxyacetamid und andere toxikologisch bedenklich sind. Hinsichtlich Polyvinylpyrrolidon hat das Bundesgesundheitsamt inzwischen sogar die Hersteller von Injektionslösungen aufgefordert, dieses aus den Injektionslosungen zu eliminieren.

Der Erfindung liegt deshalb die Aufgabe zugrunde, ein Verfahren zur Herstellung von rein wäßrigen sowie etwa isotonischen, wäßrigen Nitroglycerinlösungen zu schaffen, die nicht nur frei von organischen Lösungsmitteln, sondern auch frei von Lösungsvermittlern sind, aber dennoch eine Nitroglycerinkonzentration in dem vorteilhaften Bereich von etwa 1 mg/ml Lösung aufweisen.

Zur Lösung dieser Aufgabe wird ein Verfahren zur Herstellung einer Nitroglycerinlösung, die frei von Lösungsvermittlern und organischen Lösungsmitteln ist, gemäß den Patentansprüchen 1 bis 5 vorgeschlagen.

Wenn man Nitroglycerin auf einem festen Träger in Wasser einträgt, kann örtlich eine hohe Nitroglycerinkonzentration auftreten, die über seiner Sättigungskonzentration liegt. Das Nitroglycerin sammelt sich in Tröpfchen bzw. Tropfen, so dass solange Explosionsgefahr besteht, wie sich die Nitroglycerintropfen langsam in der überstehenden Lösung auflösen. Dementsprechend wurde bisher angenommen, dass sich dieses Vorgehen für die Herstellung von Nitroglycerinlösungen in technischem Massstab verbietet. Es wurde nun jedoch überraschend gefunden, dass man erfindungsgemäss rein wässrige und etwa isotonische, wässrige Nitroglycerinlösungen herstellen kann, wenn man eine Zubereitung aus Nitroglycerin und einem festen Träger in der erwähnten Weise in die zur Erzielung der gewünschten Nitroglycerinkonzentration erforderlichen Menge Wasser bei einer Temperatur von 90 bis 100°C und vorzugsweise 95 bis 100°C einrührt. Es hat sich nämlich überraschend gezeigt, dass bei dieser Vorgehensweise, bei der auf einen Lösungsvermittler verzichtet werden kann, keine örtlichen Übersättigungen von Nitroglycerin auftreten und damit eine Explosionsgefahr vermieden wird.

Die zu verwendende Menge an wasserlöslichem Träger ergänzt die Gesamtmenge aller Stoffe einer 0,1 g Nitroglycerin enthaltenden Zubereitungsmenge auf das etwa 0,90 g Natriumchlorid entsprechende Isotonie-Äquivalent. Das Isotonie-Äquivalent garantiert, dass man beim Auflösen einer etwa 0,1 g Nitroglycerin enthaltenden Zubereitungsmenge zu einer wässrigen Lösung mit einem Gehalt von etwa 1 mg Nitroglycerin/mg Lösung eine etwa isotonische Nitroglycerinlösung erhält. Zur Herstellung isotonischer Lösungen ist es auf dem Arzneimittelsektor üblich, die Menge der einzelnen Arzneimittelbestandteile in Natriumchlorid-Äquivalenten auszudrücken; vgl. z.B. Gstirner, Grundstoffe und Verfahren der Arzneibereitung, Stuttgart 1960, Seite 204, Tabelle 24. Bei dem 0,90 g Natriumchlorid entsprechenden Isotonie-Äquivalent werden Abweichungen toleriert, die im Bereich von 0,60 bis 1,50 g Natriumchlorid liegen oder die zu einer Gefrierpunktserniedrigung im Bereich von 0,51 bis 0,63°C führen, wenn man eine erfindungsgemässe Zubereitungsmenge mit einem Gehalt an etwa 0,1 g Nitroglycerin zu einer etwa isotonischen wässrigen Nitroglycerinlösung einer Konzentration von etwa 1 mg Nitroglycerin/ml Lösung auflöst.

Wie eingangs erwähnt, sind Nitroglycerinlösungen mit einer Konzentration von 1 mg/ml Lösung besonders vorteilhaft. Dabei sind Überschreitungen und Unterschreitungen bis zu 10%, vorzugsweise bis zu 5% und insbesondere bis zu 2% tolerierbar. Selbstverständlich können erfindungsgemäss auch etwa isotonische, wässrige Nitroglycerinlösungen hergestellt werden, die eine abweichende, aber gegenüber dem Stand der Technik sehr viel höhere Nitroglycerinkonzentration in Abwesenheit von organischen Lösungsmitteln und Lösungsvermittlern aufweisen. So sollen erfindungsgemäss auch etwa isotonische, wässrige Nitroglycerinlösungen mit einer Nitroglycerinkonzentration von 0,8 bis 1,1 mg umfasst sein.

Als Nitroglycerinträger kommen feste Stoffe in Betracht, die üblicherweise zur Herstellung von Basis-Infusionslösungen bzw. isotonen Grundlösungen verwendet werden. Beispiele für derartige Lösungen sind eine 0,9%-ige Kochsalzlösung, 5%-ige Glukoselösung, Ringerlösung, Ringerlactatlösung und Lösungen von Vollelektrolyten und deren Gemische. Beispiele für Nitroglycerinträger sind insbesondere Natriumchlorid, Mannit, Sorbit, Glukose, Lactose und Lävulose und deren Gemische; vgl. Ahnefeld & Schmitz, Systematisierung von Infusionslösungen und Grundlagen der Infusionstherapie, Karger-Verlag 1980.

Eine besonders vorteilhafte Ausführungsform der Erfindung betrifft rein wässrige Nitroglycerinlösungen, d. h. wässrige Nitroglycerinlösungen, die nicht nur frei von Lösungsvermittlern und organischen Lösungsmitteln sind, sondern auch keinen ihnen Isotonie verleihenden Stoff enthalten. Zu derartigen Nitroglycerinlösungen gelangt man, wenn man eine Zubereitung aus Nitroglycerin und einem wasserunlöslichen, festen Träger erfindungsgemäss in Wasser einrührt. Nach Auflösung des Nitroglycerins wird der wasserunlösliche Träger in üblicher Weise wie durch Filtrieren, Dekantieren etc. von der Nitroglycerinlösung abgetrennt. Als wasserunlösliche feste Träger eignen sich beispielsweise Kieselsäure, mikrokristalline Cellulose u.ä. Wenngleich die Nitroglycerinkonzentration nicht von ausschlaggebender Bedeutung ist, werden üblicherweise Zubereitungen verwendet, die etwa 2 bis 5% Nitroglycerin enthalten. Zur Herstellung von etwa isotonischen Nitroglycerinlösungen setzt man nach Auflösung des Nitroglycerins und vor oder nach der Abtrennung des wasserunlöslichen festen Trägers einen für isotonische Grundlösungen üblichen Stoff zu. Hierzu sei auf die vorangegangenen Absätze verwiesen. Besonders vorteilhaft ist die Verwendung von Natriumchlorid. Die erfindungsgemässen, rein wässrigen Nitroglycerinlösungen bieten erhebliche zusätzliche Vorteile. So bereitet die Handhabung (insbesondere Lagerung und Transport) von Zubereitungen aus Nitroglycerin und dem bevorzugten Nitroglycerinträger Natriumchlorid in der Praxis erhebliche Schwierigkeiten, da bei Zubereitungen mit der für die Herstellung von isotonischen Lösungen erforderlichen Nitroglycerinkonzentration erhebliche Explosionsgefahr besteht. Dementsprechend werden Zubereitungen auf Basis Natriumchlorid mit der erforderlichen hohen Nitroglycerinkonzentration von den entsprechenden Herstellern nicht angeboten. Andererseits wird Natriumchlorid als Isotonie verleihender Stoff für Infusionslösungen bevorzugt, da Natriumchlorid chemisch inaktiv ist und bei gleichzeitiger Infusion von Nitroglycerinlösung und anderen Wirkstofflösungen mit letzteren keine chemische Reaktion eingeht, d.h. diese nicht in unerwünschter Weise verändert, was bei den anderen oben genannten Stoffen, bei denen es sich hauptsächlich um Zucker handelt, und die dementsprechend reduzierend wirken, nicht ausgeschlossen werden kann. Hinzu kommt, dass beispielsweise Glucose enthaltende, isotonische Nitroglycerinlösungen bei Diabetikern nicht angewendet werden sollten, so dass Natriumchlorid enthaltende, isotonische Nitroglycerinlösungen erforderlich sind. Schliesslich bieten rein wässrige Nitroglycerinlösungen, wie leicht ersichtlich, den Vorteil, dass je nach Anwendungszweck und nach Kombination mit anderen Präparaten der geeignete Isotonie verleihende Stoff kurzfristig ausgewählt und durch einfaches Auflösen zugesetzt werden kann.

Die zur Herstellung der Nitroglycerinlösung zu verwendende Zubereitung kann man beispielsweise in bekannter Weise dadurch herstellen, dass man das Nitroglycerin und den Nitroglycerinträger in einem flüchtigen Lösungsmittel mischt und danach das Lösungsmittel abdampft. Bei Verwendung eines wasserlöslichen Trägers zur Herstellung einer etwa isotonischen Lösung ist das Mengenverhältnis von Nitroglycerin und Nitroglycerinträger dabei selbstverständlich so zu wählen, dass sich nach Auflösung in Wasser eine etwa isotonische Lösung ergibt. Das erfindungsgemässe Vorgehen, d.h. das Einrühren bei 90 bis 100°C garantiert, dass sich die Zubereitung in technischem Massstab ohne Ausfällung von Nitroglycerin zu einer rein wässrigen oder einer etwa isotonischen wässrigen Nitroglycerinlösung mit einer Konzentration von etwa 1 mg Glycerin/ml Lösung auflösen lässt. Wenn die Zubereitung eine negative Lösungswärme aufweist, was vom verwendeten Träger abhängt, empfiehlt es sich, die Zubereitung portionsweise unter weiterer Erwärmung einzurühren, um ein Abkühlen der Lösung zu vermeiden.

Während das Mengenverhältnis von Nitroglycerin und festem Nitroglycerinträger bei Verwendung wasserlöslicher Nitroglycerinträger durch die angestrebte Konzentration dieses Trägers in der fertigen Nitroglycerinlösung (vorzugsweise im wesentlichen isotonische Nitroglycerinlösung) festgelegt ist, besteht ein derartiger Zusammenhang bei Verwendung eines wasserunlöslichen Nitroglycerinträgers nicht. Es ist jedoch zu beachten, dass die Nitroglycerin-Konzentration auf dem Träger nicht zu hoch ist, da sonst das Risiko lokaler Überkonzentrationen und dementsprechend ein Explosionsrisiko besteht. Andererseits sind zu geringe Nitroglycerinkonzentrationen auf dem Träger aus praktischen und wirtschaftlichen Gründen nachteilig, da dann eine grosse Trägermenge abfiltriert werden muss, was mit zunehmenden Trägermengen zu immer grösseren Schwierigkeiten führt. Hierbei ist natürlich zu beachten, dass verschiedene Träger verschiedene Filtriereigenschaften aufweisen. Entsprechend kann das Mengenverhältnis von Nitroglycerin zu festem wasserunlöslichen Nitroglycerinträger, insbesondere in Abhängigkeit von den Eigenschaften des Trägers, in weiten Bereichen schwanken. Allgemein kann jedoch gesagt werden, dass die Nitroglycerinkonzentration auf dem Träger nicht oberhalb 10 Gew.-% liegen sollte. Die untere Grenze für die Nitroglycerinkonzentration auf dem wasserunlöslichen Träger richtet sich hauptsächlich nach den Eigenschaften des Trägers und kann so gering sein, wie es unter praktischen und wirtschaftlichen Gesichtspunkten noch akzeptabel ist.

Die erfindungsgemässe Lösung ist gebrauchsfertig und braucht nicht weiter verdünnt zu werden. Dadurch werden eine Kontaminationsgefahr beim Verdünnen sowie eine mögliche Inkompatibilität zwischen Wirkstoff und Infusionslösung vermieden; vgl. Ahnefeld & Schmitz, loc. cit., Seite 67 bzw. 73. Vom toxikologischen Standpunkt ist die völlige Freiheit der erfindungsgemässen. Lösung von organischen Lösungsmitteln und Lösungsvermittlern besonders vorteilhaft.

Häufig wird die erfindungsgemässe Lösung aus Stabilitätsgründen auf einen schwach sauren pH-Wert eingestellt (verbesserte Stabilität während Lagerung und Verwendung). Diese Massnahme ist bekannt und kann durch Zugabe einer Säure wie HCl zum Wasser während der Herstellung der Nitroglycerinlösung oder zur fertigen Nitroglycerinlösung erfolgen.

Im folgenden soll die Erfindung anhand eines Beispiels erläutert werden.

### Beispiel 1

Es wurde ein Ansatz aus 0,025 kg Nitroglycerin und 1,25 kg Glukose, der in der oben angegebenen Weise hergestellt worden war, unter kräftigem Rühren (Flügelrührer) in 23,75 kg Wasser, dessen pH-Wert mit HCl auf 4,7 eingestellt worden war, eingerührt, wobei die Temperatur 95°C betrug. Bei langsamer Zugabe über einen Zeitraum von etwa 5 Minuten in Portionen von etwa 0,3 kg Nitroglycerin-Glucose-Mischung kam es zu keiner Nitroglycerinausfällung. Nach Abkühlung der Lösung wurde diese in 25 ml-Ampullen oder Stechkappenflaschen abgefüllt.

### Beispiel 2

30 ml Wasser, die mit HCl auf einen pH-Wert von 4,7 angesäuert waren, wurden in einem 100 ml Becherglas auf eine Temperatur von 95 bis 100°C erhitzt. Dann wurden 0,75g mikrokristalline Cellulose (Avicel Typ PH 101 von der Firma Lehmann & Voss, Hamburg), die 4 Gew.-% Nitroglycerin enthielten (diese Zusammensetzung aus mikrokristalliner Cellulose und Nitroglycerin ist von der Firma Dynamit Nobel erhältlich), unter Rühren in einer Portion zugegeben. Nach Auflösung des Nitroglycerins wurde das Rühren abgebrochen, so dass sich der feste Träger am Boden des Becherglases absetzte. Der feste Träger wurde dann mittels eines Teflonfilters (Schleicher & Schüll 0,2 µ Filter) abfiltriert. Die erhaltene wässrige Lösung enthielt 0,8 mg Nitroglycerin/ml Lösung (theoretisch 1 mg). Etwas Nitroglycerin ging offensichtlich bei der Filtration verloren.

### Beispiel 3

Beispiel 2 wurde wiederholt mit dem Unterschied, dass anstelle der Nitroglycerin enthaltenden mikrokristallinen Cellulose Kieselsäure (Celite J2 von Dynamit Nobel) verwendet wurde, die ebenfalls 4 Gew.-% Nitroglycerin enthielt (diese Kieselsäure und Nitroglycerin enthaltene Zusammensetzung ist ebenfalls von Dynamit Nobel erhältlich). Die Konzentration der schliesslich erhaltenen Nitroglycerinlösung war wiederum etwas niedriger als der theoretische Wert.

## Patentansprüche

1. Rein wäßrige Nitroglycerinlösung, **dadurch gekennzeichnet,** daß sie aus nur Wasser und 0,8 bis 1,1 mg Nitroglycerin/ml Lösung besteht.

2. Rein wäßrige Nitroglycerinlösung nach Anspruch 1, **dadurch gekennzeichnet,** daß sie 1 mg Nitroglycerin/ml Lösung enthält.

3. Verfahren zur Herstellung einer Nitroglycerinlösung, die frei von Lösungsvermittlern und organischen Lösungsmitteln ist, **dadurch gekennzeichnet,** daß man eine Zubereitung aus Nitroglycerin und festem Nitroglycerinträger in die erforderliche Menge Wasser zur Erzielung einer Nitroglycerinkonzentration von 0,8 bis 1,1 mg und vorzugsweise 1 mg/ml Lösung bei einer Temperatur von 90 bis 100°C und vorzugsweise 95 bis 100° einrührt.

4. Verfahren nach Anspruch 3, dadurch gekennzeichnet, daß man eine Zubereitung aus Nitroglycerin und einem festen, wasserunloslichen Träger verwendet und nach Auflösung des Nitroglycerins den festen Träger in üblicher Weise abtrennt.

5. Verfahren nach Anspruch 4, dadurch gekennzeichnet, daß man nach der Auflösung des Nitroglycerins vor oder nach dem Abtrennen des festen, wasserunlöslichen Trägers einen für isotonische Grundlösungen üblichen, der Nitroglycerinlösung Isotonie verleihenden Stoff zusetzt.

6. Verfahren nach Anspruch 5, dadurch gekennzeichnet, daß man als für isotonische Grundlosungen üblichen, der Nitroglycerinlösung Isotonie verleihenden Stoff Natriumchlorid verwendet.

7. Verfahren nach Anspruch 3, dadurch gekennzeichnet, daß man eine Zubereitung aus Nitroglycerin und einem für isotonische Grundlösungen üblichen Stoff als festen Träger, die das Nitroglycerin und den festen Träger in dem zur Erzielung einer isotonischen Lösung erforderlichen Mengenverhältnis enthält, verwendet.

## Claims

1. Purely aqueous nitro-glycerine solution, characterised in that it consists of only water and 0.8 to 1.1 mg nitro-glycerine/ml of solution.

2. Purely aqueous nitro-glycerine solution according to claim 1, characterised in that it contains 1 mg of nitro-glycerine/ml of solution.

3. Process for the manufacture of a nitro-glycerine solution, which is free from solubilizers and organic solvents, characterized in that a preparation of nitro-glycerine and a solid nitro-glycerine carrier is mixed into the required quantity of water to obtain a nitro-glycerine concentration from 0.8 to 1.1 mg and preferably 1 mg/ml of solution at a temperature from 90 to 100°C and preferably from 95 to 100°C.

4. Process according to claim 3, characterised in that a preparation of nitro-glycerine and a solid, water-insoluble carrier is used and after the nitroglycerin has dissolved the solid carrier is separated in the usual way.

5. Process according to claim 4, characterised in that after the dissolution of the nitro-glycerine and before or after the separation of the solid, water-insoluble carrier a substance is added which is commonly used for isotonic stock solutions and imparts isotonicity to the nitro-glycerine solution.

6. Process according to claim 5, characterised in that sodium chloride is used as the substance commonly used for isotonic stock solutions which imparts isotonicity to the nitro-glycerine solution.

7. Process according to claim 3, characterised in that one uses a preparation of nitro-glycerine and, for isotonic stock solutions, which (preparation) contains the nitro-glycerine and the solid carrier in the quantity ratio necessary to obtain an isotonic solution.

## Revendications

1. Solution pure aqueuse de nitroglycérine, caractérisée en ce qu'elle contient seulement de l'eau et de 0,8 à 1,1 mg de nitroglycérine par ml de solution.

2. Solution pure aqueuse de nitroglycérine selon la revendication 1, caractérisée en ce qu'elle contient 1 mg de nitroglycérine par ml de solution.

3. Procédé pour la préparation d'une solution de nitroglycérine qui est exempt d'agents solubilisants et de solvants organiques, caractérisé en ce qu'on délaye dans la quantité d'eau requise une préparation composée de nitroglycérine et d'un support solide pour nitroglycérine, à une température de 90°C à 100°C, et de préférence de 95°C à 100°C, pour l'obtention d'une concentration de nitroglycérine de 0,8 à 1,1 mg et de préférence de 1 mg par ml de solution.

4. Procédé selon la revendication 3, caractérisé en ce que l'on utilise une préparation composée de nitroglycérine et d'un support solide insoluble dans l'eau, et après dissolution de la nitroglycérine, on sépare à la manière usuelle le support solide.

5. Procédé selon la revendication 4, caractérisé en ce qu'après la dissolution de la nitroglycérine, ayant ou après la séparation du support solide insoluble dans l'eau, on ajoute une substance usuelle pour solutions isotoniques de base, conférant l'isotonie à la solution de nitroglycérine.

6. Procédé selon la revendication 5, caractérisé en ce que l'on utilise du chlorure de sodium en tant que substance usuelle pour solutions isotoniques de base, conférant l'isotonie à la solution de nitroglycérine.

7. Procédé selon la revendication 3, caractérisé en ce que l'on utilise une préparation composée de nitroglycérine et, en tant que support solide, d'une substance usuelle pour solutions isotoniques de base, préparation qui contient là nitroglycérine et le support solide en la proportion requise pour l'obtention d'une solution isotonique.
